# EUROPÄISCHE PATENTSCHRIFT

(11) **EP 3 429 468 B1**
(45) Veröffentlichungstag und Bekanntmachung des Hinweises auf die Patenterteilung: **29.01.2020**
(21) Anmeldenummer: 17710530.1
(22) Anmeldetag: 13.03.2017
(51) Int. Cl.: A61B 5/0478, A61N 5/06, B82Y 5/00

(54) **MIKROELEKTRODENARRAY UND VERFAHREN ZUR HERSTELLUNG EINES MIKROELEKTRODENARRAYS**
MICRO-ELECTRODE ARRAY AND METHOD FOR PRODUCING A MICRO-ELECTRODE ARRAY
RÉSEAU DE MICRO-ÉLECTRODES ET PROCÉDÉ DE PRODUCTION D'UN RÉSEAU DE MICRO-ÉLECTRODES

(30) Priorität: 15.03.2016 DE 102016104750
(43) Veröffentlichungstag der Anmeldung: 23.01.2019
(73) Patentinhaber: Leibniz-Institut für Neurobiologie Magdeburg, 39118 Magdeburg (DE); Otto-von-Guericke-Universität Magdeburg, 39106 Magdeburg (DE)
(72) Erfinder: DECKERT, Martin, 39104 Magdeburg (DE); LIPPERT, Michael, 39104 Magdeburg (DE); SCHMIDT, Bertram, 78052 Villingen-Schwenningen (DE); OHL, Frank, 39171 Osterweddingen (DE); DADGAR, Armin, 10555 Berlin (DE)
(74) Vertreter: Gramm, Lins & Partner Patent- und Rechtsanwälte PartGmbB
(86) Internationale Anmeldenummer: PCT/EP2017/055816
(87) Internationale Veröffentlichungsnummer: WO 2017/157839

(56) Entgegenhaltungen:
- WO-A1-2015/038974
- US-A1- 2014 350 375
- US-A1- 2015 018 901
- ERIC G. R. KIM ET AL: "3D silicon neural probe with integrated optical fibers for optogenetic modulation", LAB ON A CHIP, Bd. 15, Nr. 14, 4. Juni 2015 (2015-06-04), Seiten 2939-2949, XP055299357, ISSN: 1473-0197, DOI: 10.1039/C4LC01472C

## Beschreibung

Die Erfindung betrifft ein Mikroelektrodenarray mit einem flexiblen Substrat und einer Vielzahl von Elektroden zur elektrischen Messung von neuronaler Aktivität und zur optischen Stimulation, wobei die Elektroden auf dem Substrat angeordnet sind, von der Ebene des Substrates abragen und einen lichtleitenden, von einem Lichtemitter emittiertes Licht an eine Lichtaustrittfläche am freien Ende der jeweiligen Elektrode übertragenden Kern haben.

Die Erfindung betrifft weiterhin ein Verfahren zur Herstellung eines solchen Mikroelektrodenarrays.

Mikroelektroden werden genutzt, um z.B. im Gehirn neuronale Aktivitäten robust und einfach messen zu können. Um nicht nur die neuronale Aktivität jeweils einiger weniger Zellen zu messen, werden Elektroden in Arrays bzw. Matrizen angeordnet, die bis zu einige tausend einzelne Elektroden umfassen können. Durch die hohe Kanalzahl wird es damit möglich, von so vielen Neuronen gleichzeitig abzuleiten, dass die Daten für die Realisierung z.B. von Hirn-Computer-Schnittstellen (Brain Machine Inferfaces, BMI) genutzt werden können. Hierdurch können nicht nur neuronale Kodierungsmuster analysiert werden. Solche Mikroelektrodenarrays können auch dazu genutzt werden, Patienten die Kommunikation mit der Außenwelt zu erleichtern oder eine Interaktion zu ermöglichen.

US 2013/0046148 A1 zeigt ein Elektrodenarray mit konisch spitz zulaufenden, nadelförmigen Elektroden, die einen aus einem zentralen Schaft gebildeten optischen Wellenleiter haben. Die Außenseiten der konischen, spitz zulaufenden Elektroden ist voll umfänglich elektrisch leitend beschichtet, um mit jeder Elektrodenspitze eine neurale Aktivität zu messen.

Eine ähnliche Ausführungsform eines Mikroelektrodenarrays mit einem optisch leitenden Kern, der zur Lichtführung und elektrischen Ableitung voll umfänglich mit einer Metallschicht umgeben ist, wird in J. Zhang, F. Laiwalla, J.A. Kim, H. Urabe, R. Van Wagenen, Y.-K. Song, B.B. Connors, F. Zhang, K. Deisseroth, A.V. Nurmikko: "Integrated device for optical stimulation and spatiotemporal electrical recording of neural activity in light-sensitized brain tissue", in: J Neural Eng. 2009 October; 6(5) beschrieben.

DE 10 2012 110 358 A1 beschreibt ein Mikroelektrodenarray mit einer Vielzahl von Elektroden, die in Form einer oder mehrerer Metallisierungsebenen in einem Substrat bereitgestellt werden. Zudem sind elektrische Lichtquellen einer optischen Stimulationseinheit über die flächige Ausdehnung des Substrats verteilt an der Oberfläche des Substrats angeordnet oder in dem Substrat aufgenommen.

S. Chen, W. Pei, Q. Gui, Y. Chen, S. Zhao, H. Wang, H. Chen: "A fiber-based implantable mulit-optrode array with contiguous optical and electrical sites", in: J. Neural Eng. 10 (2013) beschreiben eine Elektrodenanordnung mit mehreren stabförmigen und am Ende spitz zulaufenden Elektroden, die einen lichtleitenden Kern und einen den Kern umgebene Schicht haben. An der Spitze ist eine Elektrodenfläche umlaufend angeordnet, die über eine elektrisch leitende Schicht um die Mantelfläche der Stiftelektrode zu einem Anschlusspunkt an einem Steckverbinder geführt wird. Die Stiftelektroden werden in das Isolierstoffgehäuse des Steckverbinders eingesetzt und dort elektrisch leitend kontaktiert.

In der US2014/0350375A1 ist eine neurale Messelektrode für ein Mikroelektrodenarray zur optischen Stimulation und elektrischen Messung von neuronaler Aktivität offenbart. Die Messelektrode kann beispielsweise in V-förmige Nuten eines Substrats des Elektrodenarrays eingeklebt sein. Die Messelektrode weist einen im Wesentlichen zylindrischen Grundkörper und eine konisch zulaufende Spitze auf, an der der Lichtaustritt für die optische Stimulation erfolgt. Die Messelektrode hat einen optischen, lichtleitenden Kern, um den herum elektrisch voneinander isolierte Messleitungen angeordnet sind. Diese Messleitungen enden in Elektrodenflächen am Umfang der Messelektrode.

Eric G.R. Kim et al: "3D silicon neural probe with integrated optical fibers for optogenetic modulation (Lab on a Chip, vol. 15, no. 14, 2015-06-04) beschreibt ein Herstellungsverfahren für ein Mikroelektrodenarray. Die hierin gezeigten Mikroelektroden weisen integrierte optische Fasern auf, die in ein Röhrchen eingebettet sind, an dessen Umfang Elektrodenflächen angeordnet sind. Die Röhrchen münden in starre Silikonspitzen am freie Ende der Mikroelektroden.

Die WO 2015/038974 A1 offenbart ein Verfahren zum Herstellen einer Mikroelektrode mit einem Elektrodensubstrat, einzeln adressierbaren Messelektrodenflächen und einer Beschichtung. Die so hergestellte Mikroelektrode weist einen Schaft und eine Messspitze auf. Die Messelektrodenflächen sind an einem Endbereich des Schafts angeordnet.

Bei der Anwendung der Elektroden wird das Gewebe in der Umgebung der Elektrodenspitzen geschädigt, so dass die neurale Aktivität in der direkten Umgebung der Elektrodenspitzen nachlässt. Zudem ist der Zusammenbau der Mikroelektrodenarrays aufwendig und schwierig.

Ausgehend hiervon ist es Aufgabe der vorliegenden Erfindung ein verbessertes Mikroelektrodenarray sowie ein verbessertes Verfahren zur Herstellung von solchen Mikroelektrodenarrays zu schaffen.

Die Aufgabe wird mit dem Mikroelektrodenarray mit den Merkmalen des Anspruchs 1 sowie durch das Verfahren mit den Merkmalen des Anspruchs 15 gelöst. Vorteilhafte Ausführungsformen sind in den Unteransprüchen beschrieben.

Für ein Mikroelektrodenarray der eingangs genannten Art wird vorgeschlagen, dass der lichtleitende Kern integral mit dem flexiblen Substrat verbunden und flexibel ist, und dass um den Umfang des lichtleitenden Kerns herum verteilt mehrere elektrisch voneinander isolierte Messleitungen angeordnet sind, die vom Substrat zu einer Grundfläche am freien Ende der jeweiligen Elektrode geführt sind. Benachbart zu der Endfläche des lichtleitenden Kerns sind auf der Grundfläche am Ende der Elektroden mehrere räumlich um die Endfläche verteilt angeordnete Elektrodenflächen vorhanden, die jeweils elektrisch leitend mit einer zugehörigen Messleitung verbunden sind. Die zugehörigen Messleitungen können in der gleichen Schichtebene und/oder in unterschiedlichen Schichtebenen im Mikroelektrodenarray integriert sein.

Um die Endfläche herum sind somit mehrere Elektroden angeordnet. Damit kann eine räumliche Messung neuraler Aktivität erfolgen. Dies ist insbesondere von Vorteil, wenn das in der direkten Umgebung der Elektrodenspitze liegende Gewebe geschädigt wird, und die neurale Aktivität von entfernteren Stellen herrührt. Mit den mehreren Elektroden pro Elektrodenspitze kann somit ein differenzierteres Signal gewonnen werden, aus dem eine Auswertung neuraler Aktivität in der Umgebung des Mikroelektrodenarrays gut möglich ist.

Durch das Vorsehen mehrerer Messstellen ist die Kontaktierung selbiger mit einzelnen Messleitungen nötig, welche als möglichst schmale Streifenleiter ausgeführt sind. Dadurch reduziert sich die immense Kapazität bisheriger konzentrischer Metallisierungen zum umgebenden Gewebe, was nun die Messung einzelner Aktionspotentiale erlaubt. Auch die Trennung der räumlich verteilt gemessenen Signale der einzelnen Elektrodenöffnungen wird möglich, da sie sich kaum noch intereinander und zum Gewebe hin beeinflussen.
Der Kern stellt mit seinem hohen Aspektverhältnis, d.h. einer größeren Länge als Dicke (Durchmesser) einen Träger für die Elektroden und zugehörigen Messleitungen bereit. Er kann zudem bei Bedarf in Form eines lichtleitenden Kerns mit einer Lichtaustrittsfläche als Endfläche als Wellenleiter für elektromagnetische Wellen im sichtbaren und/oder unsichtbaren Wellenlängenbereich (z.B. Infrarot, Ultraviolett, Terahertz) genutzt werden, um Zellen zu stimulieren.

Anders als das einfache herkömmliche Beschichten der Mantelfläche einer konisch zulaufenden Elektrode wird durch die Anordnung mehrerer Messleitungen an der Mantelfläche an der Außenfläche des Kerns erreicht, dass das elektrische Verhalten verbessert wird.

Hierzu sind die Messleitungen um den Umfang der Außenfläche des Kerns herum verteilt angeordnet. Zudem kann ein Multilayeraufbau die elektromagnetische Schirmung einzelner Messleitungen voneinander erlauben.

Wenn anstelle von konzentrischen oder teilkonzentrischen elektrisch leitenden Schichten zur Bildung von Messleitungen auf die Mantelfläche der Kerne direkt Leiterbahnen strukturiert werden, reduziert sich die Kapazität der leitenden Fläche zum umgebenden Gewebe hin. Da die Impedanz der Elektrodenkontakte relativ hoch ist, führen die bei der konzentrischen Beschichtung der Mantelfläche der Kerne auftretenden parasitären Kapazitäten zu einer starken Dämpfung hochfrequenter Signale, insbesondere der Signale der Aktionspotentiale der Zellen. Dies wird durch die Aufbringung einer Mehrzahl von elektrisch voneinander isolierten Messleitungen an der Außenfläche des Kerns vermieden. Zumindest werden die parasitären Kapazitäten wesentlich verringert.

Alternativ zu einfachen Ableitelektroden können zur Detektion schwacher elektrischer Signale bzw. elektrostatischer Potentiale Feldeffekttransitoren eingesetzt werden. Diese erfordern im Gegensatz zu einfachen Ableitelektroden sowohl eine Zuleitung als auch eine Ableitung, verstärken aber direkt das gemessene Signal und können somit zu einer empfindlicheren und rauschärmeren Detektion beitragen. Durch eine angelegte Spannung an diesen Leitungen kann das zur Neuronenumgebung zwischen den Kontakten offene System durch eine Potentialänderung die Leitfähigkeit und damit den fließenden Strom verändern. Dabei wirkt die offene Fläche zwischen den beiden Kontakten ganz oder zum großen Teil als Gateelektrode. Vorzugsweise kommen hier Transistoren auf der Basis polarer Halbleiter zum Einsatz, da diese eine besonders hohe Empfindlichkeit aufweisen. Hier sind Feldeffekttransistoren auf der Basis von Verbindungshalbleitern aus den Gruppe-III-Nitriden, insbesondere GaN sehr gut geeignet. Dabei weist GaN eine hohe Biokompatibilität und Langzeitstabilität in lebenden Organismen auf. Der Gatebereich kann aber auch zusätzlich durch Beschichtungen in seinen Eigenschaften modifiziert und die Empfindlichkeit damit beeinflusst werden. Insbesondere lässt sie sich durch hochisolierende Schichten wie z.B. Siliziumdioxid oder Siliziumnitrid elektrisch sehr gut von der Neuronenumgebung isolieren um einen möglichen Einfluss zu minimieren.

Der Kern kann zylinderförmig sein und eine Grundfläche an seinem freien Ende haben, auf dessen Ebene die zentrale Endfläche (z.B. die Lichtaustrittfläche eines lichtleitenden Kerns) und mehrere um den Umfang der zentralen Endfläche herum verteilt angeordnete Elektrodenflächen angeordnet sind. Anstelle von spitz zulaufenden konischen Elektroden werden somit zylinderförmige Säulenkerne vorgeschlagen. Diese lassen sich aufgrund der Unterschnittfreien aber nahezu senkrecht (95° ± 5°) zur Ebene des Substrats stehenden Mantelflächen der lichtleitenden Kerne adäquat zur Ausbildung von Messleitungen an der Mantelfläche strukturieren. Zudem lassen sich solche zylinderförmigen Säulenkerne gut im Schichtaufbau integral in dem flexiblen Substrat herstellen, wodurch ein einstückiges flexibles Mikroelektrodenarray geschaffen wird, das beim Einbringen in Gewebe einen wesentlich reduzierten zerstörerischen Einfluss auf das Gewebe hat. Zudem wird durch die Grundfläche des zylinderförmigen lichtleitenden Kerns ein Flächenbereich um die zentrale Endfläche herum bereitgestellt, auf der Elektrodenflächen aufgebracht werden können. Diese Grundfläche kann eben, leicht gekrümmt oder geneigt sein. Vorteilhaft ist jedoch, wenn die Grundfläche nicht spitz zulaufend ist, um die Gefahr der Zerstörung von Gewebe zu verringern.

Das flexible Substrat ist vorzugsweise polymerhaltig. Es kann beispielsweise aus einem Polyimid gebildet werden, so dass eine progressive Implantation einzelner Elektroden aufgrund der hohen Flexibilität möglich ist. Hierdurch können Gewebeschäden reduziert und die Blutzufuhr weniger beeinträchtigt werden, da der bei der Implantation auftretende Druck reduziert werden kann. Zudem lassen sich in eine solche flexible Polymerstruktur Lichtemitter, wie beispielsweise Leuchtdioden, sowie elektrische Leitungen einfach und gut einbauen.

Der Kern kann aus einem Polymethylacrylat (PMMA) gebildet sein, das sich auch gut als Lichtleiter eignet. Sowohl das polymerhaltige Substrat, als auch der PMMA-Säulenkern können lithographisch gut strukturiert werden, um Elektrodenleitungen und Elektrodenflächen auszubilden. Das polymerhaltige Substrat kann sich gut mit dem PMMA-Säulenkern integral verbinden, wenn der Säulenkern schichtweise durch Aufbringen von PMMA-Material auf das Substrat ausgebildet wird. Des Weiteren kann der Säulenkern auch plattenartig aus einem Stück aufgebaut, direkt gebondet und lithographisch strukturiert werden. In allen Ausführungsformen ist der PMMA-Säulenkern dabei selbst noch so flexibel, dass eine Gewebeschädigung hierdurch reduziert wird.

Der Kern kann oberflächenbehandelt sein. Hierbei ist es vorteilhaft durch Strukturierung der Mantelfläche eines lichtleitenden Kerns, beispielsweise den Brechungsindex an der Außenseite des lichtleitenden Kerns anzupassen. Durch eine Reduzierung des Brechungsindexes ("Gradientenindex") an der Außenfläche des lichtleitenden Substrats wird die Lichtführung im lichtleitenden Kern (Wellenleiter) verbessert, ohne dass eine spiegelnde metallische Beschichtung an der Außenseite des lichtleitenden Kerns erforderlich ist, welche bislang zudem als Messleitung genutzt wird. Denkbar ist auch eine Beschichtung des lichtleitenden Kerns durch ein zweites durchsichtiges Material mit einem niedrigeren Brechungsindex (Step-Index) als der lichtleitende Kern. Die Messleitungen müssen damit nicht mehr als Teil des optischen Systems genutzt werden, sondern können ausschließlich im Hinblick auf ihre elektrische Funktion, z.B. in schmalen Bahnen an der Außenseite des lichtleitenden Kerns strukturiert werden. Damit lässt sich die Messung von Aktionspotentialen verbessern.

Eine Verbesserung des Lichtemitters, insbesondere ein kleineres Lichtanregungsvolumen und damit eine gezieltere Anregung der Neuronen, lässt sich durch die Verwendung stark gerichteter Lichtquellen deutlich besser erreichen als mit einem in den gesamten Halbraum abstrahlenden Emitter. Hierfür eignen sich als Erweiterung einfacher LEDs sogenannte Resonant Cavity LEDs (RCLED), welche meist einen dielektrischen oder monolitischen Braggspiegel aufweisen. Dadurch ist die Emission aus der Oberfläche der LED stark gerichtet. Eine weitere Verbesserung lässt sich durch vertikal emittierende Laser (Vertical Cavity Surface Emitting Laser, VCSEL) erzielen, bei denen zusätzlich ein zweiter Braggspiegel über der RCLED zu einer stark gerichteten kohärenten Lichtemission mit geringem Öffnungswinkel führt, womit sich, je nach Bauelementdurchmesser, Lichtstrahlen im Nahfeld mit Durchmessern unter 10 µm und geringem Öffnungswinkel realisieren lassen. Damit lassen sich prinzipiell z. B. einzelne Neuronen, bzw. Neuronen-Populationen mit hoher Genauigkeit adressieren. Ein Vorteil solcher Lichtemitter ist zudem, obwohl sie klein ausgelegt sind, dass sich mit ihnen trotzdem eine hohe Lichtleistung in einem kleinen Volumen erzielen lässt und sie zum anderen nur eine geringe Leistung aufnehmen was zu einer Vereinfachung der Ansteuerung führt, da zum einen die Leitungen dünn sind, und zum anderen die als Wärme mit eingebrachte Verlustleistung gering ist.

Prinzipiell kann eine gute Fokussierung des Lichts auch durch eine veränderte Ausgestaltung des lichtleitenden Kerns bewerkstelligt werden. Hier gibt es mehrere Möglichkeiten, dies zu erzielen. Zum einen kann eine Wölbung des Lichtaustritts bzw. die Ausgestaltung der Zylinderendfläche als Linse zu einer Kollimation des Lichtstrahls führen. Bei Kenntnis der Eindringtiefe und des ungefähren Abstands der zu adressierenden Neuronen, lässt die Fokussierung eine gezielte Adressierung der Neuronen zu, da der Strahl bei kurzer Brennweite nur in einem kleinen Tiefenbereich bzw. Abstand von der Lichtaustrittsöffnung eine ausreichende Intensität zum Anregen eines Neurons bzw. einer Gruppe von Neuronen aufweist.

Eine andere Verbesserungsmöglichkeit ergibt sich durch eine Strukturierung der Oberfläche des lichtleitenden Zylinders durch Einbringen bzw. Aufbringen von Dickenmodulationen oder aufgedampften Materialien wie Metallen oder anderen Dielektrika als Lichtleiter in regelmäßigen Abständen. Dies bewirkt, ähnlich wie ein Braggspiegel, die Reflektion eines Wellenlängenbands, welches so gewählt werden kann, dass nur ein Wellenlängenbereich des von der LED emittierten Lichts eine hohe Transmission durch den Wellenleiter aufweist, womit die insgesamt auf die Neuronen eingestrahlte Leistung verringert wird und, bei breitbandigeren Quellen, nur die für die Anregung geeignete Wellenlänge in das Gewebe eindringt. Prinzipiell kann der Lichtwellenleiter ganz oder teilweise auch als oberer Spiegel für einen VCSEL genutzt werden. So besteht in diesem Fall die Lichtquelle aus einer RC-LED mit untenliegendem Spiegel und der Lichtwellenleiter der durch eins der obigen Verfahren als oberer Spiegel, nun in Resonanz mit dem unteren Spiegel und der Kavität in der die lichtemittierende Schicht sitzt. Durch gerichtete Lichtquellen lässt sich aufgrund der höheren Lichtintensität in Emissionsrichtung die Bauelementgröße deutlich verkleinern und die Packungsdichte solcher einzeln adressierbarer Lichtquellen auf dem Träger deutlich erhöhen, womit eine genauere Adressierung einzelner Neuronen bzw. kleiner Gruppen von Neuronen möglich ist und einfacher komplexe Anregungsmuster übertragen werden können.

Die Mantelfläche des Kerns kann zur Ausbildung der Messleitung und Elektrodenflächen lithographisch strukturiert werden. Vorzugsweise sind Lichtemitter von dem Substrat getragen, die jeweils angrenzend an den Übergang eines lichtleitenden Kerns zu dem Substrat angeordnet sind. Durch den Aufbau oder Einbau eines Lichtemitters, wie beispielsweise einer Leuchtdiode im Übergang zwischen Substrat und lichtleitendem Kern wird das von dem Lichtemitter emittierte Licht nahezu verlustlos über den lichtleitenden Kern an die Lichtaustrittfläche am freien Ende der jeweiligen Elektrode übertragen.

Die Elektroden des Mikroelektrodenarrays können in unterschiedlichen Längen ausgebildet sein, um unterschiedlich tiefe kortikale Bereiche zu erreichen. Dabei ist auch denkbar, dass Elektroden und/oder Lichtemitter auf der Substratoberfläche vorhanden sind (d.h. die Höhe der Elektroden ist Null). Damit kann eine planare Messung mit einer dreidimensionalen Messung neuronaler Aktivität kombiniert werden.

Die Herstellung des Mikroelektrodenarrays gelingt dadurch, dass ein Material auf ein flexibles Substrat schichtweise aufgetragen wird oder als plattenförmigen Materialschicht direkt auf ein flexibles Substrat gebondet wird, um integrale Elektroden mit flexiblen säulenförmigen lichtleitenden Kernen zu bilden. Diese Kerne können beispielsweise als lichtleitende Kerne auf oder in dem Substrat angeordneten Lichtemittern (beispielsweise Leuchtdioden) direkt aufgebracht werden und verbinden sich integral mit dem flexiblen Substrat. Anschließend erfolgt ein Einlaminierungsschritt der Kerne und/oder die direkte Ausbildung einer Mehrzahl von Messleitungen an der Mantelfläche der Kerne durch lithographische Strukturierung. Diese lithographische Strukturierung kann direkt auf dem Material des Kerns vorgenommen werden oder auf einer Beschichtung des Kerns oder auf einem dünnen Polymerstrukturlayer (Laminationslayer), welche/r dann jeweils die Mantelfläche bildet. Für die lithographische Strukturierung kann beispielsweise ein fotoempfindlicher Lack auf die Mantelfläche der Kerne aufgebracht werden und nach Belichtung und Entfernung der weniger oder mehr belichteten Bereiche durch Sputtern, Bedampfen oder Ähnliches mit elektrisch leitendem Material befüllt werden, um die Mehrzahl von Messleitungen in dem Vergleich zum Umfang der Mantelfläche reduzierter Breite sowie ggf. auch die Elektrodenflächen in beispielsweise einem Lift-off Prozess auszubilden.

Es ist eine mehrschichtige Metallisierung der im Wesentlichen zylinderförmigen Säulenstrukturen von oben, d.h. von der dem Substrat gegenüberliegenden Seite möglich. Dies erlaubt die Verwendung von Standardprozessen auf Si-Wafern, ohne dass Glaswafer zur Strukturierung der Kerne von unten, d.h. durch das Substrat hindurch benötigt werden.

Auf diese Weise wird auf einfache Weise ein monolithisches Mikroelektrodenarray geschaffen, das benachbart zur Endfläche des Kerns eine Mehrzahl von Elektrodenflächen hat, die über jeweils zugeordnete Messleitungen mit einer Messeinheit bzw. Auswerteeinheit verbunden werden können.

Das Mikroelektrodenarray sollte mit einem polymerhaltigen Material, wie z.B. Polyimid, so passiviert sein, dass nur die Elektroden mit ihren Elektrodenflächen neuronales Gewebe elektrisch kontaktieren.

Die Erfindung wird nachfolgend anhand eines Ausführungsbeispiels mit den beigefügten Zeichnungen näher erläutert: Es zeigen:
- Fig. 1 -: perspektivische Skizze eines Mikroelektrodenarrays;
- Fig. 2 -: Ausschnittsansicht des Mikroelektrodenarrays aus Fig. 1 mit einer auf einem Substrat angeordneten Elektrode;
- Fig. 3 -: Skizze eines auf in ein Gewebe eingebrachtes Mikroelektrodenarray, das mit einer Auswerteeinheit verbunden ist mit beispielhaften Elektrodensignalen mit und ohne optische Stimulation.

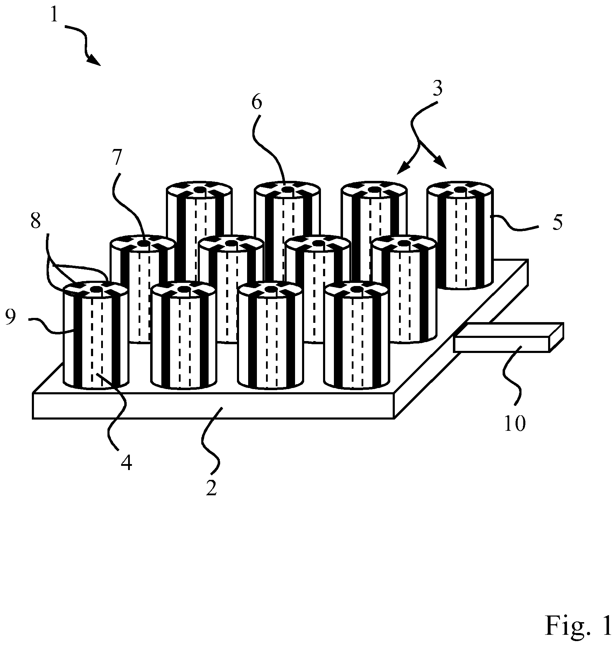

Figur 1 lässt eine perspektivische Skizze eines Mikroelektrodenarrays 1 erkennen, das ein flexibles Substrat 2 hat. Das flexible Substrat 2 ist beispielsweise aus einem Polymer ausgebildet und damit relativ weich und bei der Implantierung des Mikroelektrodenarrays1 in Gewebe nachgiebig. Geeignet ist z.B. Polyimid als Grundmaterial zur Herstellung des Substrats 2.

In oder auf das Substrat können optional Lichtemitter befestigt sein (nicht sichtbar). Auf dem Substrat 2, optional über jeweils einem Lichtemitter, werden dann Elektroden 3 schichtweise aufgebaut. Die Elektroden 3 sind in einer Matrix bevorzugt mit gleichen Abständen reihenweise im Abstand zueinander angeordnet. Die Elektroden 3 haben einen auf das Substrat 2 schichtweise oder monolithisch aufgebrachten Kern 4, der aus einem anderen Material als eine den Kern 4 umgebende Schichtstruktur 5 besteht, beispielsweise PMMA oder SU8. Diese Schichtstruktur 5 bildet zusammen mit dem Kern 4 eine Elektrodensäule, die in etwa zylinderförmig ist und eine Grundfläche 6 hat. Erkennbar ist, dass die Elektroden 3 an der Grundfläche 6 nicht spitz zulaufen und die Elektrodenwände nahezu senkrecht zur Ebene des darunterliegenden Substrats 2 stehen. Sie können auch leicht geneigt sein (im Bereich von etwa 0 bis 5 Grad, ohne Unterschnitt), sollten jedoch nicht kegelförmig sein. Der Kern 4 kann aus einem lichtleitenden Material gebildet sein, um als Wellenleiter für sichtbares und/oder unsichtbares Licht für die Stimulation von Zellen zu dienen.

An der Grundfläche 6 ist eine zentrale Endfläche 7 des Kerns 4 vorhanden. Diese Endfläche 7 bildet für die z.B. lichtleitenden Kerne 4 eine Lichtaustrittsfläche. Weiterhin sind auf der Grundfläche 6 Elektrodenflächen 8 im Umfang um die zentrale Endfläche 7 herum verteilt angeordnet. Diese Elektrodenflächen 8 sind elektrisch leitend mit jeweils einer zugeordneten Messleitung 9 verbunden, die an der Mantelfläche der Elektroden zum Substrat 2 hingeführt sind. In dem Substrat 2 sind dann die Messleitungen 8 zu einer Anschlussleitung 10 hingeführt, über die dann eine Auswerteeinheit angeschlossen werden kann.

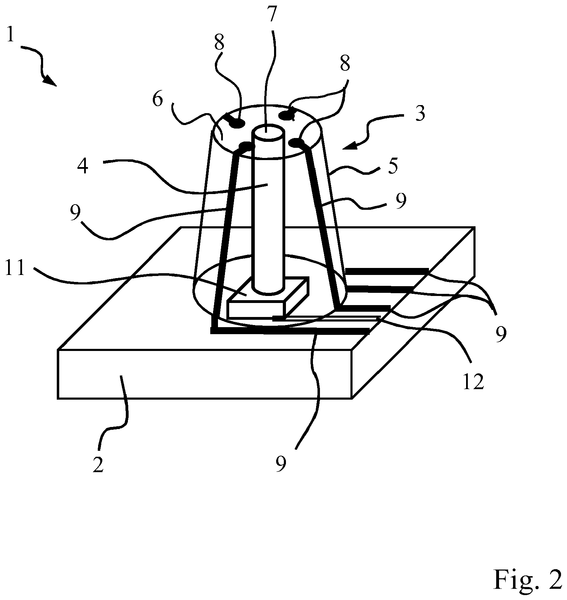

Figur 2 lässt eine Skizze eines Ausschnitts eines Mikroelektrodenarrays 1 aus Figur 1 erkennen. Deutlich wird nun, dass das Substrat 2 einen Lichtemitter 11, z.B. eine Leuchtdiode tragen kann, die über eine Steuerleitung 12 wahlweise angesteuert werden kann, um Lichtsignale abzugeben und damit Gewebe optisch zu stimulieren. Das vom Lichtemitter 11 emittierte Licht wird dann über den lichtleitenden Kern 4 an der zentralen Endfläche 7 (Lichtaustrittsfläche) abgestrahlt. Die nach einer solchen optischen Stimulation resultierende neurale Aktivität kann dann mit Hilfe der Elektrodenflächen 8 gemessen werden, die räumlich um die Endfläche 7 herum angeordnet sind. Über die Anordnung von Elektrodenflächen 8 auf der Grundfläche 6 hinaus können auch noch weitere Elektrodenflächen 8 am Umfang der Mantelfläche der Elektrode 3 vorhanden sein.

Die den Kern 4 umgebende Schichtstruktur 5 ist nun so ausgebildet, dass Messleitungen 9 an der Mantelfläche von der Grundfläche 6 der Elektrode 3 zur Struktur 2 hin geführt sind. Die Messleitungen 9 sind dabei elektrisch voneinander isoliert und über den Umfang verteilt angeordnet. Sie können, anders als skizziert, auch in den Kern 4 eingearbeitet sein, oder in eine Zwischenschicht, die dann von einer elektrisch isolierenden Schicht umgeben ist, welche dann die Außenfläche der Elektrode 3 bildet.

Deutlich wird, das die Messleitungen 9 dann auf der Oberfläche des Substrats 2 oder in dem Substrat 2 z.B. parallel zur Ebene des Substrats 2 an einer Randkante des Substrats 2 geführt werden, um dort zusammengefasst in eine Auswerteeinheit führbar zu sein. Das Zusammenfassen der Messleitungen 9 kann auch in einem Steckverbinder erfolgen, an das dann ein Messkabel einer Auswerteeinheit angeklemmt wird.

Der Kern 4 kann beispielsweise aus Polymethylacrylat PMMA gebildet werden. Die den Kern 4 umgebende Schichtstruktur 5 kann wiederum aus dem flexiblen Material des Substrats 2, wie beispielsweise Polyimid gebildet sein. Denkbar ist aber auch eine mikrotechnisch aufgebrachte weiche Außenbeschichtung, die z.B. aus Parylene oder Polydimethylsiloxan (PDMS) bestehen kann. Hierdurch kann in Verbindung mit der intrinsischen Flexibilität der Polymerstruktur des Substrats 2 die Immunantwort weiter reduziert werden.

Die lichtleitenden Kerne 4 haben einen höheren Brechungsindex (Gradientenindex) als das umgebende Strukturmaterial 5. Dies kann auch durch unterschiedliche Materialien oder durch Oberflächenbearbeitung der Mantelfläche des lichtleitenden Kerns 4 sichergestellt werden. Durch die Verringerung des Brechungsindexes des lichtleitenden Kerns 4 an seiner Säulenaußenfläche (Mantelfläche) oder durch Beschichtung der Mantelfläche des lichtleitenden Kerns 4 mit einem zweiten durchsichtigen Material mit niedrigerem Brechungsindex (Step-Index) wird eine gute Lichtführung mit möglichst geringen Lichtverlusten erreicht. Das von dem Lichtemitter 11 abgestrahlte Licht wird dann weitestgehend nur an der zentralen Lichtaustrittsfläche 7 emittiert.

Durch die Strukturierung der Messleitungen 9 als Leiterbahnen auf der Mantelfläche der Elektrode 3 wird im Vergleich zur konzentrischen oder teilkonzentrischen Ausbildung von Messleitungen 9 die Kapazität der leitenden Fläche zum umgebenden Gewebe erheblich reduziert. Aufgrund der hohen Impedanz der Elektrodenflächen 8 (Elektrodenkontakte) sind hohe parasitäre Kapazitäten vor allem für hochfrequente Signale störend, wie sie bei Aktionspotentialen der Zellen auftreten.

Die Bereitstellung mehrerer elektrophysiologisch ableitender Elektrodenflächen 8 verteilt um die Endfläche 7 herum erlaubt mit Hilfe von Triangulationsverfahren die Ableitung bzw. Unterscheidung tieferliegender Signalquellen, d.h. von neuronaler Aktivität. Dies ist insbesondere wichtig, wenn um die Elektroden 3 direkt herum liegendes Gewebe durch das Einbringen des Mikroelektrodenarrays 1 geschädigt wurde. Dadurch, dass die Elektrodenflächen 8 punktförmig oder rechteckig, jedenfalls nicht konzentrisch sind, gelingt eine Reduktion signaldegradierender Effekte durch parasitäre Kapazitäten, die aufgrund einer konzentrischen Komplettmetallisierung entstehen würden. Durch die Reduktion der Breite der Messleitungen 9, d.h. der Zuleitungen über den Umfang der säulenförmigen Elektroden 3 werden die parasitären Kapazitäten weiter verringert, die auf dieser Leiterbahn entlang der säulenförmigen Elektroden 3, d.h. auf unterschiedlichen Mantelflächen der Elektrode 3 mit voneinander unterschiedlichen Radien aufgebracht werden.

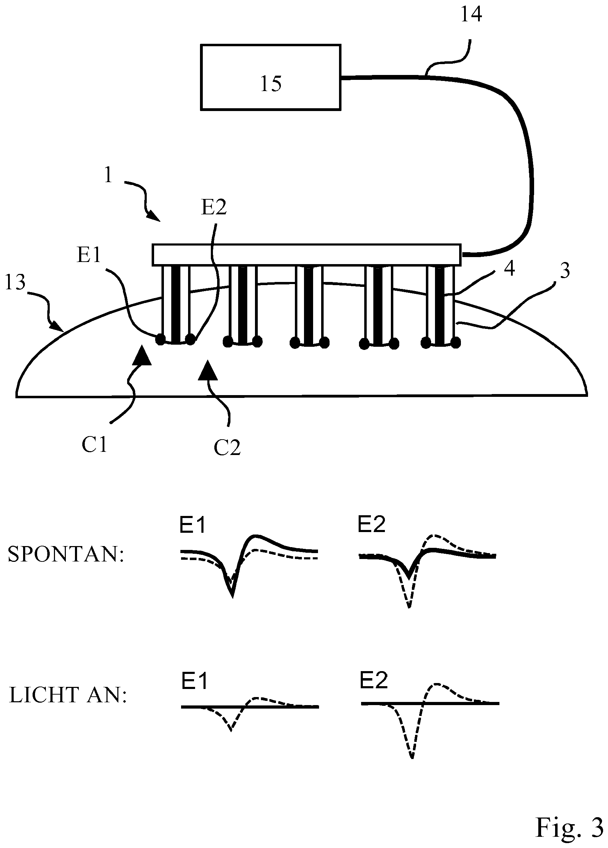

Figur 3 lässt eine Skizze des Mikroelektrodenarrays 1 aus Figur 1 in einem Zustand erkennen, bei dem es in ein Gewebe 13, beispielsweise eine Gehirns, implantiert ist. Das Mikroelektrodenarray 1 hat an seinen Elektroden 3 um die Endfläche 7 des Kerns 4 herum verteilte Elektroden 8, von denen beispielhaft zwei Elektroden E1, E2 skizziert sind. Deutlich wird, dass das Mikroelektrodenarray 1 über ein Messkabel 14 mit einer Auswerteeinheit 15 verbunden ist. Die Auswerteeinheit 15 kann geeignete Messverstärker haben und ist auch zur wahlweisen Ansteuerung der Lichtemitter 11 eingerichtet.

Neuronen C1, C2 des Gewebes 13 sind beispielhaft skizziert.

Für eine spontane Aktivität ergeben sich beispielsweise die von den Elektroden E1 und E2 aufgenommenen Signale der Nervenzellen C1 und C2. Die schwarze Linie ist dabei die von der zur Elektronenfläche E1 näher liegende Aktivität der Nervenzelle C1. Die gestrichelte Linie ist die der zweiten Elektrodenfläche E2 näher liegende spontane Aktivität der Nervenzelle C2.

Erkennbar ist, dass die Elektrodenfläche E1 in Aktivität der nähergelegenen Nervenzelle C2 der höheren und die Aktivität der entfernteren Nervenzelle C2 mit einer niedrigeren Amplitude aufzeichnet. Für die zweite Elektrode E2 ist das Signalverhältnis entsprechend umgekehrt.

Wenn es sich bei der zweiten Nervenzelle C2 um eine mit einem exzitatorischen optogenetischen Opsin lichtsensitiv gemachten Zelle handelt, so antwortet nur diese Nervenzelle C2 auf Lichtimpulse aus dem lichtleitenden Kern 4 (Licht an).

Die Nervenzelle C1 zeigt dann keine Aktivität (durchgezogene schwarze Linie für die Aktivität der Nervenzelle C1).

Durch räumliche Selektivität der nahe beieinander liegenden Elektrodenflächen E1, E2, von denen deshalb immer mindestens zwei Elektrodenflächen 8 pro Elektrode 3 vorhanden sein sollten, ergibt sich eine bessere Trennungsmöglichkeit von Signalen unterschiedlicher Nervenzellen.

Die Herstellung des Mikroelektrodenarrays 1 erfolgt mit einem Verfahren mit den Schritten:
a) Auftragen von Material auf ein flexibles Substrat 2 zur Bildung von integralen säulenförmigen Elektroden 3 mit flexiblen lichtleitenden Kernen 4, und
b) lithographisches Strukturerzeugung auf den Mantelflächen der jeweiligen säulenförmigen Elektrode 3, um an dem Kern 4 oder einen den Kern 4 umgebenden Mantelfläche eine Mehrzahl von Messleitungen 9 auszubilden, die vom Substrat 2 zu einer Grundfläche 6 am freien Ende der jeweiligen Elektrode 3 geführt sind, um den Umfang des lichtleitenden Kerns 4 herum verteilt angeordnet sind und elektrisch voneinander isoliert sind, und von Elektrodenflächen 8, die mit jeweils einer zugeordneten Messleitung 9 verbunden werden, wobei die Elektrodenflächen 9 räumlich um die Endfläche 7 des jeweiligen Kerns 4 verteilt angeordnet sind,
c) Passivierung der gebildeten Gesamtstruktur unter Offenhaltung oder Öffnung der Elektrodenflächen.

Die Passivierung der Oberfläche der Gesamtstruktur kann z.B. durch Beschichtung mit einem Polymer, wie z.B. Polyimid erfolgen. Die Elektrodenflächen 8, E1, E2 können dabei durch Abdeckung offengehalten werden, um eine zur Außenseite frei zugängliche Fläche bereitzustellen. Es kann aber auch nach der Beschichtung eine z.B. Lithographie basierte Öffnung der Elektroden 3 durch beispielsweise ein Trockenätzverfahren erfolgen.

Die lithographische Strukturierung kann mit Fotolacken (z.B. SU8 und Poly-Acryl) als Material für den Kern 4 erfolgen. Denkbar ist aber auch eine Herstellung mit Druck- oder Gießverfahren zum Aufbau der säulenförmigen Elektroden 3 und insbesondere der Kerne 4, möglichst auch ein 3D-Rapid Prototype-Prozess zur schichtweisen Ausbildung der säulenförmigen Elektroden 3. Durch den Kern 4 kann eine Penetration des Gehirngewebes orthogonal zur Fläche des Mikroelektrodenarrays 1 erfolgen, und es ist nicht nötig, Teile des Mikroelektrodenarrays 1 umzufalten. Die Mikroelektrodenarrays 1 werden in einem solchen Mikrofabrikationsprozess monolithisch hergestellt.

Die Elektroden 3 selbst können als Öffnungen auf der Grundfläche 6 oder der Mantelfläche der Elektroden 3 realisiert werden, an die sich eine elektrisch leitende Elektrodenfläche 8 anschließt.

Bei einem Mikroelektrodenarray 1 ist auch denkbar, dass einzelne Elektroden 3 nicht zur optischen Stimulation ausgebildet sind, aber einen stabilisierenden Kern vergleichbar zu dem lichtleitenden Kern 4 haben.

Die Elektroden 3 können in unterschiedlichen Längen ausgebildet sein, um unterschiedlich tiefe kortikale Bereiche zu erreichen.

## Patentansprüche

1. Mikroelektrodenarray (1) mit einem flexiblen Substrat (2) und einer Vielzahl von Elektroden (3) zur elektrischen Messung neuronaler Aktivitäten, wobei die Elektroden (3) auf dem Substrat (2) angeordnet sind, von der Ebene des Substrats (2) abragen und einen lichtleitenden, von einem Lichtemitter emittiertes Licht an eine Lichtaustrittfläche am freien Ende der jeweiligen Elektrode übertragenden Kern (4) haben, wobei der lichtleitende Kern (4) integral mit dem flexiblen Substrat (2) verbunden und flexibel ist, und um den Umfang des lichtleitenden Kerns (4) herum verteilt mehrere elektrisch voneinander isolierte Messleitungen (9) angeordnet sind, die vom Substrat (2) zu einer Grundfläche (6) des lichtleitenden Kerns (4) am freien Ende der jeweiligen Elektrode (3) geführt sind, wobei an dem Ende der Elektroden (3) auf der Ebene der Grundfläche (6) mehrere räumlich um den Umfang einer zentralen Endfläche (7) verteilt angeordnete Elektrodenflächen (8) sind, die jeweils elektrisch leitend mit einer zugehörigen Messleitung (9) verbunden sind, wobei die zentrale Endfläche (7) die Lichtaustrittsfläche des lichtleitenden Kerns (4) bildet.

2. Mikroelektrodenarray (1) nach Anspruch 1, **dadurch gekennzeichnet, dass** der lichtleitende Kern (4) zylinderförmig ist und auf der Ebene seiner Grundfläche (6) die zentrale Endfläche (7) und die um den Umfang der zentralen Endfläche (7) herum verteilt angeordneten Elektrodenflächen (8) angeordnet sind.

3. Mikroelektrodenarray (1) nach Anspruch 1 oder 2, **dadurch gekennzeichnet, dass** das Substrat (2) polymerhaltig ist.

4. Mikroelektrodenarray (1) nach einem der vorhergehenden Ansprüche, **dadurch gekennzeichnet, dass** der lichtleitende Kern (4) aus Polymethylacrylat (PMMA) gebildet ist.

5. Mikroelektrodenarray (1) nach einem der vorhergehenden Ansprüche, **dadurch gekennzeichnet, dass** der lichtleitende Kern (4) oberflächenbehandelt ist.

6. Mikroelektrodenarray (1) nach einem der vorhergehenden Ansprüche, **dadurch gekennzeichnet, dass** die Mantelfläche des lichtleitenden Kerns (4) oder einer den lichtleitenden Kern (4) umgebenden Schichtstruktur (5) zur Ausbildung der Messleitungen (9) und Elektrodenflächen (8) lithographisch strukturiert ist.

7. Mikroelektrodenarray (1) nach einem der vorhergehenden Ansprüche, **dadurch gekennzeichnet, dass** die Lichtaustrittsfläche eine Wölbung aufweist.

8. Mikroelektrodenarray (1) nach einem der vorhergehenden Ansprüche, **dadurch gekennzeichnet, dass** der Brechungsindex der Mantelfläche des lichtleitenden Kerns (4) größer als der Brechungsindex einer den lichtleitenden Kern (4) umgebenden Schicht ist.

9. Mikroelektrodenarray (1) nach einem der vorhergehenden Ansprüche, **dadurch gekennzeichnet, dass** Lichtemitter (11) von dem Substrat (2) getragen werden, die jeweils angrenzend an den Übergang eines lichtleitenden Kerns (4) zu dem Substrat (2) angeordnet sind.

10. Mikroelektrodenarray (1) nach einem der vorhergehenden Ansprüche, **dadurch gekennzeichnet, dass** Elektroden (3) mit unterschiedlicher Länge voneinander vorgesehen sind.

11. Mikroelektrodenarray (1) nach einem der vorhergehenden Ansprüche, **dadurch gekennzeichnet, dass** der Lichtemitter als Leuchtdiode mit einem das emittierte Licht reflektierenden Spiegel ausgebildet ist.

12. Mikroelektrodenarray (1) nach einem der vorhergehenden Ansprüche, **dadurch gekennzeichnet, dass** der Lichtemitter als oberflächenemittierender Laser ausgebildet ist.

13. Mikroelektrodenarray (1) nach einem der vorhergehenden Ansprüche, **dadurch gekennzeichnet, dass** die Elektroden Feldeffekttransistoren aufweisen.

14. Mikroelektrodenarray (1) nach Anspruch 13, **dadurch gekennzeichnet, dass** die Feldeffekttransistoren aus einem polaren Material ausgebildet sind, wobei die Feldeffekttransistoren einen Source-Kontakt, einen Drain-Kontakt und einen Gate-Kontakt haben, und die zwischen dem Source-Kontakt und Drain-Kontakt liegende Fläche ganz oder teilweise als Gate-Kontakt ausgelegt ist, wobei der Gate-Kontakt in Kontakt mit der zu messenden Umgebung steht.

15. Verfahren zur monolithischen Herstellung eines Mikroelektrodenarrays (1) nach einem der vorhergehenden Ansprüche mit den Schritten:
- Auftragen von Material auf ein flexibles Substrat (2) zur Bildung von integralen säulenförmigen Elektroden (3) mit flexiblen lichtleitenden Kernen (4);
- lithographisches Strukturieren einer Mantelfläche der jeweiligen säulenförmigen Elektrode (3) zur Ausbildung einer Mehrzahl von Messleitungen (9), die vom Substrat (2) zu einer Grundfläche (6) am freien Ende der jeweiligen Elektrode (3) geführt sind, um den Umfang des lichtleitenden Kerns (4) herum verteilt angeordnet sind und die elektrisch voneinander isoliert sind, und zur Ausbildung von Elektrodenflächen (8), die mit jeweils einer zugehörigen Messleitung (9) verbunden werden,
wobei die Elektrodenflächen (8) räumlich um die Endfläche (7) des jeweiligen lichtleitenden Kerns (4) verteilt angeordnet werden.

16. Verfahren nach Anspruch 15, **gekennzeichnet durch** Oberflächenbehandlung der Mantelflächen der lichtleitend ausgeführten Kerne (4) derart, dass der Brechungsindex an den Mantelflächen der lichtleitenden Kerne (4) kleiner als der Brechungsindex der lichtleitenden Kerne (4) ist.

17. Verfahren nach Anspruch 15 oder 16, **gekennzeichnet durch** schichtweisen Aufbau der Kerne (4) auf das flexible Substrat (2).

18. Verfahren nach einem der Ansprüche 15 bis 17, **gekennzeichnet durch** Passivierung der gebildeten Gesamtstruktur und Öffnung der Elektrodenflächen (8).

19. Verfahren nach Anspruch 18, **dadurch gekennzeichnet, dass** die Passivierung durch Beschichtung mit einem Polymer, insbesondere mit Polyimid, und die Öffnung der Elektrodenflächen (8) lithographisch, insbesondere mit einem Trockenätzprozess erfolgt.

## Claims

1. Micro-electrode array (1) comprising a flexible substrate (2) and a multiplicity of electrodes (3) for electrically measuring neural activities, wherein the electrodes (3) are arranged on the substrate (2), project from the plane of the substrate (2) and have a light guiding core (4) that transmits light emitted by a light emitter to a light exit surface at the free end of the respective electrode, wherein the light guiding core (4) is integrally connected to the flexible substrate (2) and is flexible, and a plurality of measurement lines (9) that are electrically insulated from one another are arranged in a manner distributed around the circumference of the light guiding core (4), said measurement lines being led from the substrate (2) to a base surface (6) of the light guiding core (4) at the free end of the respective electrode (3), wherein at the end of the electrodes (3) on the plane of the base surface (6) there are a plurality of electrode surfaces (8) arranged in a manner distributed spatially around the circumference of a central end surface (7), said electrode surfaces in each case being electrically conductively connected to an associated measurement line (9), wherein the central end surface (7) forms the light exit surface of the light guiding core (4).

2. Micro-electrode array (1) according to Claim 1, **characterized in that** the light guiding core (4) is cylindrical and the central end surface (7) and the electrode surfaces (8) arranged in a manner distributed around the circumference of the central end surface (7) are arranged on the plane of the base surface (6) of said light guiding core.

3. Micro-electrode array (1) according to Claim 1 or 2, **characterized in that** the substrate (2) is polymer-containing.

4. Micro-electrode array (1) according to any of the preceding claims, **characterized in that** the light guiding core (4) is formed from polymethyl acrylate (PMMA).

5. Micro-electrode array (1) according to any of the preceding claims, **characterized in that** the light guiding core (4) is surface-treated.

6. Micro-electrode array (1) according to any of the preceding claims, **characterized in that** the lateral surface of the light guiding core (4) or of a layer structure (5) surrounding the light guiding core (4) is lithographically patterned for forming the measurement lines (9) and electrode surfaces (8).

7. Micro-electrode array (1) according to any of the preceding claims, **characterized in that** the light exit surface has a curvature.

8. Micro-electrode array (1) according to any of the preceding claims, **characterized in that** the refractive index of the lateral surface of the light guiding core (4) is greater than the refractive index of a layer surrounding the light guiding core (4).

9. Micro-electrode array (1) according to any of the preceding claims, **characterized in that** light emitters (11) are carried by the substrate (2), said light emitters being arranged in each case in a manner adjoining the transition from a light guiding core (4) to the substrate (2).

10. Micro-electrode array (1) according to any of the preceding claims, **characterized in that** electrodes (3) are provided with mutually different lengths.

11. Micro-electrode array (1) according to any of the preceding claims, **characterized in that** the light emitter is configured as a light emitting diode with a mirror that reflects the emitted light.

12. Micro-electrode array (1) according to any of the preceding claims, **characterized in that** the light emitter is configured as a surface emitting laser.

13. Micro-electrode array (1) according to any of the preceding claims, **characterized in that** the electrodes comprise field effect transistors.

14. Micro-electrode array (1) according to Claim 13, **characterized in that** the field effect transistors are formed from a polar material, wherein the field effect transistors have a source contact, a drain contact and a gate contact, and the surface situated between the source contact and drain contact is designed wholly or partly as a gate contact, wherein the gate contact is in contact with the environment to be measured.

15. Method for monolithically producing a micro-electrode array (1) according to any of the preceding claims, comprising the following steps:
- applying material on a flexible substrate (2) for the purpose of forming integral columnar electrodes (3) with flexible light guiding cores (4);
- lithographically patterning a lateral surface of the respective columnar electrode (3) for the purpose of forming a plurality of measurement lines (9), which are led from the substrate (2) to a base surface (6) at the free end of the respective electrode (3), are arranged in a manner distributed around the circumference of the light guiding core (4) and which are electrically insulated from one another, and for the purpose of forming electrode surfaces (8), which are connected respectively to an associated measurement line (9),
wherein the electrode surfaces (8) are arranged in a manner distributed spatially around the end surface (7) of the respective light guiding core (4).

16. Method according to Claim 15, **characterized by** surface treatment of the lateral surfaces of the cores (4) embodied in light guiding fashion in such a way that the refractive index at the lateral surfaces of the light guiding cores (4) is less than the refractive index of the light guiding cores (4).

17. Method according to Claim 15 or 16, **characterized by** layered build-up of the cores (4) on the flexible substrate (2).

18. Method according to any of Claims 15 to 17, **characterized by** passivation of the overall structure formed and opening of the electrode surfaces (8).

19. Method according to Claim 18, **characterized in that** the passivation is carried out by coating with a polymer, in particular with polyimide, and the opening of the electrode surfaces (8) is carried out lithographically, in particular by a dry etching process.

## Revendications

1. Réseau de microélectrodes (1) muni d'un substrat flexible (2) et d'une pluralité d'électrodes (3) pour la mesure électrique d'activités neuronales, dans lequel les électrodes (3) sont disposées sur le substrat (2), dépassent du plan du substrat (2) et présentent un noyau conducteur de lumière (4) transmettant la lumière émise par un émetteur de lumière au niveau d'une surface de sortie de lumière à l'extrémité libre de l'électrode respective, dans lequel le noyau conducteur de lumière (4) est relié de manière intégrale au substrat flexible (2) et est flexible, et plusieurs lignes de mesure (9) isolées électriquement les unes des autres sont disposées de manière répartie sur la périphérie du noyau conducteur de lumière (4), et sont guidées depuis le substrat (2) vers une surface de base (6) du noyau conducteur de lumière (4) à une extrémité libre de l'électrode respective (3), dans lequel plusieurs surfaces d'électrodes (8) sont disposées de manière répartie spatialement sur la périphérie d'une surface d'extrémité centrale (7) à l'extrémité des électrodes (3) sur le plan de la surface de base (6) et sont respectivement reliées de manière électriquement conductrice à une ligne de mesure (9) correspondante, dans lequel la surface d'extrémité centrale (7) forme la surface de sortie de lumière du noyau conducteur de lumière (4).

2. Réseau de microélectrodes (1) selon la revendication 1, **caractérisé en ce que** le noyau conducteur de lumière (4) est de forme cylindrique et **en ce que** la surface d'extrémité centrale (7) et les surfaces d'électrodes (8) disposées de manière répartie sur la périphérie de la surface d'extrémité centrale (7) sont disposées sur le plan de sa surface de base (6).

3. Réseau de microélectrodes (1) selon la revendication 1 ou 2, **caractérisé en ce que** le substrat (2) contient un polymère.

4. Réseau de microélectrodes (1) selon l'une des revendications précédentes, **caractérisé en ce que** le noyau conducteur de lumière (4) est formé de polyacrylate de méthyle (PMMA).

5. Réseau de microélectrodes (1) selon l'une des revendications précédentes, **caractérisé en ce que** le noyau conducteur de lumière (4) est traité en surface.

6. Réseau de microélectrodes (1) selon l'une des revendications précédentes, **caractérisé en ce que** la surface de revêtement du noyau conducteur de lumière (4) ou d'une structure en couches (5) entourant le noyau conducteur de lumière (4) est structurée de manière lithographique pour réaliser les lignes de mesure (9) et les surfaces d'électrodes (8).

7. Réseau de microélectrodes (1) selon l'une des revendications précédentes, **caractérisé en ce que** la surface de sortie de lumière présente une forme bombée.

8. Réseau de microélectrodes (1) selon l'une des revendications précédentes, **caractérisé en ce que** l'indice de réfraction de la surface de revêtement du noyau conducteur de lumière (4) est supérieur à l'indice de réfraction d'une couche entourant le noyau conducteur de lumière (4).

9. Réseau de microélectrodes (1) selon l'une des revendications précédentes, **caractérisé en ce que** des émetteurs de lumière (11) sont portés par le substrat (2) et sont disposés de manière adjacente à la transition entre un noyau conducteur de lumière (4) et le substrat (2).

10. Réseau de microélectrodes (1) selon l'une des revendications précédentes, **caractérisé en ce qu'**il est prévu des électrodes (3) ayant des longueurs différentes les unes des autres.

11. Réseau de microélectrodes (1) selon l'une des revendications précédentes, **caractérisé en ce que** l'émetteur de lumière est réalisé sous la forme d'une diode électroluminescente munie d'un miroir réfléchissant la lumière émise.

12. Réseau de microélectrodes (1) selon l'une des revendications précédentes, **caractérisé en ce que** l'émetteur de lumière est réalisé sous la forme d'un laser à émission de surface.

13. Réseau de microélectrodes (1) selon l'une des revendications précédentes, **caractérisé en ce que** les électrodes comportent des transistors à effet de champ.

14. Réseau de microélectrodes (1) selon la revendication 13, **caractérisé en ce que** les transistors à effet de champ sont réalisés à partir d'un matériau polaire, dans lequel les transistors à effet de champ présentent un contact de source, un contact de drain et un contact de grille, et la surface située entre le contact de source et le contact de drain est en tout ou partie réalisée sous la forme d'un contact de grille, dans lequel le contact de grille est en contact avec l'environnement à mesurer.

15. Procédé de fabrication monolithique d'un réseau de microélectrodes (1) selon l'une des revendications précédentes, comprenant les étapes suivantes :
- application d'un matériau sur un substrat flexible (2) pour former des électrodes cylindriques intégrales (3) munies de noyaux conducteurs de lumière (4) flexibles ;
- structuration lithographique d'une surface de revêtement de l'électrode en forme de colonne (3) respective pour réaliser une pluralité de lignes de mesure (9) qui sont guidées depuis le substrat (2) vers une surface de base (6) à une extrémité libre de l'électrode (3) respective, qui sont disposées de manière répartie sur la périphérie du noyau conducteur de lumière (4) et qui sont électriquement isolées les unes des autres et sont respectivement reliées à une ligne de mesure (9) correspondante pour réaliser des surfaces d'électrodes (8), dans lequel les surfaces d'électrodes (8) sont disposées de manière répartie spatialement sur la périphérie de la surface d'extrémité (7) du noyau conducteur de lumière (4) respectif.

16. Procédé selon la revendication 15, **caractérisé par** un traitement de surface des surfaces de revêtement des noyaux conducteurs de lumière (4) de manière à ce que l'indice de réfraction au niveau des surfaces de revêtement des noyaux conducteurs de lumière (4) soit inférieur à l'indice de réfraction des noyaux conducteurs de lumière (4).

17. Procédé selon la revendication 15 ou 16, **caractérisé par** une structure en couches des noyaux (4) sur le substrat flexible (2).

18. Procédé selon l'une des revendications 15 à 17, **caractérisé par** la passivation de l'ensemble de la structure formée et par l'ouverture des surfaces d'électrodes (8).

19. Procédé selon la revendication 18, **caractérisé en ce que** la passivation est effectuée par revêtement avec un polymère, en particulier avec un polyimide, et **en ce que** l'ouverture des surfaces d'électrodes (8) s'effectue de manière lithographique, en particulier par un procédé de gravure sèche.
